# EUROPÄISCHE PATENTSCHRIFT

(11) **EP 1 483 008 B1**
(45) Veröffentlichungstag und Bekanntmachung des Hinweises auf die Patenterteilung: **20.02.2008**
(21) Anmeldenummer: 03743827.2
(22) Anmeldetag: 28.02.2003
(51) Int. Cl.: A61M 25/00

(54) **SKLEROSIERUNGSKATHETER ZUR SKLEROSIERUNG VON BLUTGEFÄSSEN INSBESONDERE VENEN**
SCLEROSING CATHETER FOR SCLEROSING BLOOD VESSELS, ESPECIALLY VEINS
CATHETER SCLEROSANT DESTINE A SCLEROSER DES VAISSEAUX SANGUINS, EN PARTICULIER DES VEINES

(30) Priorität: 09.03.2002 DE 20203840 U; 25.07.2002 DE 10233794
(43) Veröffentlichungstag der Anmeldung: 08.12.2004
(73) Patentinhaber: BRODERSEN, Jens P., 30175 Hannover (DE)
(72) Erfinder: BRODERSEN, Jens P., 30175 Hannover (DE)
(74) Vertreter: Wagner, Carsten
(86) Internationale Anmeldenummer: PCT/EP2003/002063
(87) Internationale Veröffentlichungsnummer: WO 2003/075993

(56) Entgegenhaltungen:
- EP-A- 0 266 928
- US-A- 4 752 286
- US-A- 5 047 013
- US-A- 5 221 255
- US-A- 5 451 206
- US-A- 6 021 340
- US-A1- 2002 010 418

## Beschreibung

Die Erfindung betrifft einen Sklerosierungskatheter zur Sklerosierung von Blutgefäßen, insbesondere Venen.

Durch DE 199 57 168 A1 ist ein zur Behandlung von Beinvenenthrombosen vorgesehener Katheter bekannt, der drei Lumina aufweist, wobei ein erstes Lumen ein mit Luft befüll- und aufblasbares erstes ballonartiges Element aufweist. Der Katheter besteht aus einem biegsamen Material und weist ein zweites Lumen auf, das zum Führen eines Lytikums dient, das zum Auflösen bei einer Thrombose auftretender Blutgerinnsel eingesetzt wird. Für den Austritt des Lytikums ist in dem zweiten Lumen eine Austrittsöffnung vorgesehen.

Durch DE 693 26 572 T2 und EP 0 557 80 B1 ist ein Hämodialyse-Katheter bekannt, der mehrere Lumina aufweist.

Durch DE 691 10 467 T2 und EP 0 533 816 B1 ist ein Katheter zur Abgabe von Medikamenten bekannt, der ein Lumen aufweist, das mit einem mit Luft befüll- und aufblasbaren ballonartigen Element in Verbindung steht. Die Abgabe des Medikamentes erfolgt über das ballonartige Element, das zu diesem Zweck Austrittsöffnungen für das Medikament aufweist. Um mittels des Katheters auch schwer zugängliche Stellen im menschlichen Körper erreichen zu können, ist der Katheter flexibel ausgebildet.

Durch DE 698 22 456 T2 und EP 0 364 799 B1 ist ein für die Chemotherapie vorgesehener Katheter bekannt, der ein erstes Lumen aufweist, das mit ballonartigen Elementen, die mit Luft befüll- und aufblasbar sind, in Verbindung steht. Der Katheter weist ferner ein zweites Lumen zur Führung eines Chemotherapeutikums auf, wobei das zweite Lumen eine Mehrzahl von Austrittsöffnungen für das Chemotherapeutikum aufweist. Die Austrittsöffnungen sind zwischen zwei in Axialrichtung des Katheters zueinander beabstandeten ballonartigen Elementen angeordnet. Abgesehen von einem zwischen den ballonartigen Elementen liegenden Bereich, in dem die Austrittsöffnungen angeordnet sind, ist der Katheter flexibel ausgebildet, um mittels des Katheters auch schwer zugängliche Stellen im menschlichen Körper erreichen zu können.

Durch WO 01/2861 A2 ist ein Thrombektomie-Katheter bekannt, der mehrere Lumina aufweist, von denen eines ein mit Luft befüll- und aufblasbares ballonartiges Element aufweist.

Durch FR 2 803 532 ist ein Arteriotomie-Katheter bekannt, der zwei in Axialrichtung zueinander beabstandete mit Luft befüll- und aufblasbare ballonartige Elemente aufweist. Um mittels des Katheters auch schwer zugängliche Stellen im menschlichen Körper zu erreichen, ist der Katheter flexibel ausgebildet.

Durch WO 02/05887 A2 ist ein Katheter zur Abgabe von Medikamenten bekannt, der ein erstes Lumen aufweist, das mit zwei mit Luft befüll- und aufblasbaren ballonartigen Elementen in Verbindung steht. Der Katheter weist ein zweites Lumen auf, das zur Abgabe eines Medikamentes dient und Austrittsöffnungen aufweist, die in Axialrichtung des Katheters zwischen den ballonartigen Elementen angeordnet sind. Um mittels des Katheters auch schwer zugängliche Stellen im menschlichen Körper erreichen zu können, ist der Katheter flexibel ausgebildet.

US 2002/0010418 A1 offenbart einen mehrlumigen Sklerosierungskatheter der betreffenden Art gemäß Oberbegriff von Anspruch 1 mit mehreren Röhren, der an einem Ende einer Röhre einen aufblasbaren Ballon aufweist. Die andere Röhre weist ein Lumen auf, das der Zuführung von Luft zum Ballon dient, um diesen aufzublasen und den Blutfluß in der Vene zu stoppen. Der Sklerosierungskatheter weist ferner einen selbstausdehnenden zweiten Ballon auf, der relativ zum ersten Ballon verschieblich angeordnet ist und mit einem ringförmigen Raum in Verbindung steht, der zwischen der ersten Röhre und einer weiteren Röhre gebildet ist, wobei der ringförmige Raum der Zuführung eines Medikamentes zum zweiten Ballon dient, der mit Austrittsöffnungen für das Medikament versehen ist. Der Sklerosierungskatheter wird zunächst in eine Vene eingeführt, wobei der erste Ballon aufgeblasen wird, sobald eine gewünschte Position erreicht ist. Anschließend wird ein äußeres Rohrteil des Sklerosierungskatheters zurückgezogen, so daß sich der selbstausdehnende zweite Ballon ausdehnt. Danach wird über den ringförmigen Raum zwischen den beiden Röhren ein Medikament in den selbstausdehnenden Ballon eingeführt, das über die in dem Ballon gebildeten Austrittsöffnungen austritt und so auf die Innenwandung des zu behandelnden Gefäßes appliziert wird. Schließlich wird der Ballon in Längsrichtung der Vene zurückgezogen, wobei das Medikament sich an der Innenwandung des Gefäßes verteilt.

Nachteilig bei dieser Ausgestaltung ist der relativ komplizierte Aufbau des Sklerosierungskatheters, insbesondere im Hinblick auf den beweglichen zweiten Ballon, der innerhalb der zu verödenden Vene in axialer Richtung verschoben werden muß, um das entsprechende Medikament an der Veneninnenwand gleichmäßig zu verteilen. Dadurch ist der bekannte Sklerosierungskatheter teuer in der Herstellung.

Das der vorliegenden Erfindung zugrundeliegende Problem besteht daher darin, die oben genannten Nachteile zu vermeiden, insbesondere einen vom Aufbau her einfachen Sklerosierungskatheter bereitzustellen, der dennoch eine gleichmäßige Applizierung eines Sklerosierungsmittels an einer Gefäßinnenwand ermöglicht.

Dieses Problem wird erfindungsgemäß durch einen Sklerosierungskatheter nach Anspruch 1 gelöst.

Der erfindungsgemäße Sklerosierungskatheter zur Sklerosierung von Blutgefäßen, insbesondere Venen, weist mindestens zwei Lumina auf. Zum Stoppen des Blutflusses weist das erste Lumen ein mit Luft befüll- und aufblasbares erstes ballonartiges Element auf. Das zweite Lumen weist zur Applizierung eines Sklerosierungsmittels mindestens eine relativ zum ersten ballonartigen Element ortsfeste Austrittsöffnung auf.

Entgegen dem eingangs erwähnten Stand der Technik ist eine in axialer Richtung verschiebliche, mit Austrittsöffnungen versehene Einrichtung zur Applizierung an einer Gefäßinnenwand nicht erforderlich, weil es sich überraschenderweise gezeigt hat, daß mindestens eine ortsfeste Austrittsöffnung vollkommen ausreichend ist, da durch den mittels des Sklerosierungsmittels ausgelösten partiell oder vollständig auftretenden Spasmus des Gefäßes bei Verringerung des Gefäßdurchmessers eine perestaltikartige Einschnürung bei gleichzeitigem Vorantreiben des Sklerosierungsmittels an noch nicht benetzten Teilen der Gefäßinnenwand auftritt. Aufgrund dieser überraschenden Erkenntnis ist eine derartig einfache konstruktive Ausgestaltung möglich.

Der Sklerosierungskatheter selbst kann als Kunststoffschlauch, beispielsweise aus Polyurethan, ausgestaltet sein, wobei die Austrittsöffnung schlitzartig oder lochförmig sein kann. Üblicherweise beträgt die Länge des Sklerosierungskatheters ca. 30 bis 100 cm bei einem Gesamtaußendurchmesser von etwa 2 bis 5 mm. Das mit Luft befüll- und aufblasbare erste ballonartige Element besteht in der Regel aus Polyurethan, Silikon oder Gummi. Im aufgeblasenen Zustand beträgt der Durchmesser des ballonartigen Elementes in der Regel 2 bis 4 cm. Üblicherweise ist die mindestens eine Austrittsöffnung zur Applizierung des Sklerosierungsmittels einige Millimeter vom ballonartigen Element beabstandet.

Erfindungsgemäß ist das zweite Lumen im wesentlichen entlang seiner gesamten Länge derart formstabil ausgebildet, daß bei Erzeugung eines zum Absaugen überschüssigen Sklerosierungsmittels aus dem Blutgefäß erforderlichen Unterdruckes in dem zweiten Lumen ein Verschließen des zweiten Lumens durch Kontakt der Innenwandungen des zweiten Lumens miteinander verhindert ist. Das Absaugen überschüssigen Sklerosierungsmittels nach Abschluß der Sklerosierung des behandelten Blutgefäßes ist wünschenswert, um zu verhindern, daß das Sklerosierungsmittel nach Abschluß der Behandlung in unerwünschter Weise im Körper des Patienten verbleibt und dort möglicherweise zu Gesundheitsschäden führt. Um das Sklerosierungsmittel abzusaugen, wird in dem zweiten Lumen ein Unterdruck erzeugt, durch den das Sklerosierungsmittel, möglicherweise vermischt mit Blut, aus dem behandelten Blutgefäß abgesaugt wird und durch die Austrittsöffnungen in das zweite Lumen des Katheters hineingesaugt wird. Bei herkömmlichen, aus besonders biegsamen Kunststoffschläuchen bestehenden Kathetern würde die Erzeugung eines zum Absaugen des Sklerosierungsmittels erforderlichen Unterdruckes dazu führen, daß sich die Wandungen des zweiten Lumens elastisch verformen, bis die gegenüberliegenden Bereiche der Innenwandungen des zweiten Lumens aneinander anliegen und so das zweite Lumen verschließen, so daß ein Absaugen des Sklerosierungsmittels aus dem Blutgefäß verhindert ist. Um ein solches Verschließen des zweiten Lumens durch elastische Verformung der Wandungen des zweiten Lumens zu verhindern, ist die Formstabilität des zweiten Lumens erfindungsgemäß so gewählt, daß bei Erzeugung eines zum Absaugen überschüssigen Sklerosierungsmittels aus dem Blutgefäß erforderlichen Unterdruckes ein Verschließen des zweiten Lumens verhindert ist.

Um ein Einführen des Katheters in das Blutgefäß auch bei gekrümmten Blutgefäßen zu ermöglichen, kann der erfindungsgemäße Katheter flexibel ausgebildet sein. Entscheidend ist, daß die Formstabilität des zweiten Lumens so groß ist, daß ein Verschließen des zweiten Lumens bei Erzeugung des zum Absaugen überschüssigen Sklerosierungsmittel erforderlichen Unterdruckes in dem zweiten Lumen verhindert ist.

Insbesondere bei Vorliegen von sog. Perforansvenen, also Verbindungsvenen zwischen dem oberflächlichen und dem tiefen Venensystem, ist es von besonderem Vorteil, wenn das erste Lumen zum weiteren Verschließen des Blutgefäßes ein relativ zur Austrittsöffnung beabstandetes, mit Luft befüll- und aufblasbares zweites ballonartiges Element aufweist, wobei die Austrittsöffnung zwischen erstem und zweitem ballonartigen Element angeordnet ist, um ein Abfließen des Sklerosierungsmittels über die Perforansvene zu verhindern. Da beide ballonartigen Elemente Teile eines Lumens sind, werden diese nahezu synchron aufgeblasen.

Eine weitere vorteilhafte Ausgestaltung besteht darin, wenn ein drittes Lumen zum weiteren Verschließen des Blutgefäßes ein relativ zur Austrittsöffnung beabstandetes, mit Luft befüll- und aufblasbares zweites ballonartiges Element aufweist, wobei die Austrittsöffnung zwischen erstem und zweitem ballonartigen Element angeordnet ist, da auf diese Weise ein unabhängiges Befüllen und Aufblasen mit Luft der beiden ballonartigen Elemente möglich ist.

Damit der in den ballonartigen Elementen vorhandene Überdruck im aufgeblasenen Zustand bequem gehalten werden kann, ohne ständig Luft nachführen zu müssen, ist es von Vorteil, wenn das erste und/oder dritte Lumen eine Luftabsperreinrichtung, insbesondere ein Ventil, Gummistopfen oder eine Quetschklemme, zum Absperren aus dem Lumen austretbarer Luft aufweist.

Üblicherweise weisen die Katheterenden, die zur Luft- und Medikamentenzuführung dienen, unterschiedliche Farbmarkierungen auf, um Verwechslungen zu vermeiden.

Eine außerordentlich vorteilhafte Weiterbildung der erfindungsgemäßen Lehre sieht vor, daß das distale Ende des Sklerosierungskatheters abgebogen oder unter einem Winkel von weniger als 45°, vorzugsweise von etwa 30° zur Längsachse des Sklerosierungskatheters, abgewinkelt ist. Bei dieser Ausführungsform ist das Einführen des Sklerosierungskatheters auch in gekrümmte Blutgefäße erleichtert. Stößt das distale Ende des Sklerosierungskatheters beim Einführen in ein Blutgefäß gegen ein Hindernis, so kann der Sklerosierungskatheter durch Verdrehen um seine Längsachse in eine Verdrehlage bewegt werden, in der das distale Ende des Sklerosierungskatheters dem Verlauf des Blutgefäßes folgt.

Grundsätzlich können die Wandungen des ersten Lumens und/oder des zweiten Lumens und/oder des dritten Lumens elastisch im wesentlichen unverformbar ausgebildet sein. Um ein Einführen des Sklerosierungskatheters in das Blutgefäß zu erleichtern, ist es zweckmäßig, daß die Wandungen des ersten Lumens und/oder des zweiten Lumens und/oder des dritten Lumens wenigstens abschnittsweise aus einem elastisch verformbaren Material bestehen. Auf diese Weise kann sich der Katheter beim Einführen in das Blutgefäß elastisch verformen, wobei jedoch das zweite Lumen im wesentlichen entlang seiner gesamten Länge derart formstabil ausgebildet ist, daß bei Erzeugung eines zum Absaugen überschüssigen Sklerosierungsmittels aus dem Blutgefäß erforderlichen Unterdrucks in dem zweiten Lumen ein Verschließen des zweiten Lumens durch elastische Verformungen der Innenwandungen des zweiten Lumens verhindert ist.

Grundsätzlich kann die erforderliche Formstabilität des zweiten Lumens dadurch erzielt werden, daß die Wandungen des zweiten Lumens aus einem entsprechend formsteifen Material bestehen. Eine vorteilhafte Weiterbildung der erfindungsgemäßen Lehre sieht Stabilisierungsmittel zur Formstabilisierung des zweiten Lumens vor. Bei dieser Ausführungsform können die Wandungen des zweiten Lumens an sich aus einem in hohem Maße elastischen Material bestehen. Die ein Verschließen des zweiten Lumens bei Erzeugung eines Unterdruckes verhindernde Formstabilität des zweiten Lumens ist bei dieser Ausführungsform durch die Stabilisierungsmittel erzielt.

Bei der vorgenannten Ausführungsform können die Stabilisierungsmittel wenigstens eine sich in Radialrichtung zwischen den Innenwandungen des zweiten Lumens erstreckende Rippe oder wenigstens einen sich in Radialrichtung zwischen den Innenwandungen des zweiten Lumens erstreckenden Steg aufweisen, wie dies eine Weiterbildung vorsieht. Eine Weiterbildung der vorgenannten Ausführungsform sieht vor, daß sich die Rippe oder die Rippen bzw. der Steg oder die Stege in Axialrichtung des zweiten Lumens im wesentlichen über die gesamte Länge des zweiten Lumens erstreckt bzw. erstrekken. Bei dieser Ausführungsform ist entlang der gesamten Länge des zweiten Lumens die gleiche Formstabilität erzielt.

Eine andere Weiterbildung der erfindungsgemäßen Lehre sieht vor, daß eine Mehrzahl von Rippen oder Stegen vorgesehen ist, die sich in Axialrichtung jeweils über ein kurzes Stück der Länge des zweiten Lumens erstrecken und die in Axialrichtung zueinander beabstandet sind. Bei dieser Ausführungsform kann an den axialen Stellen, an denen die Rippen oder Stege angeordnet sind, eine hohe Formstabilität erzielt werden, während der Katheter in den in Axialrichtung zwischen den Rippen oder Stegen liegenden Bereichen flexibel ausgebildet sein kann. Auf diese Weise ist einerseits verhindert, daß sich das zweite Lumen bei Erzeugung eines Unterdruckes durch Berührung gegenüberliegender Bereiche der Innenwandung verschließt. Andererseits ist durch die in den Bereichen zwischen den Rippen oder Stegen vorgesehene Flexibilität ein Einführen des Katheters in das Blutgefäß erleichtert.

Entsprechend den jeweiligen Anforderungen kann das zweite Lumen durch die Rippe oder die Rippen bzw. den Steg oder die Stege in wenigstens zwei Einzellumina unterteilt sein, wie dies eine Weiterbildung vorsieht.

Bei der vorgenannten Ausbildungsform können die Einzellumina voneinander getrennt sein oder miteinander kommunizieren, wie dies andere Weiterbildungen vorsehen.

Anhand der nachfolgenden Ausführungsbeispiele wird die Erfindung näher erläutert.

Dabei zeigen:
- Fig. 1 -: eine skizzenhafte Darstellung einer ersten Ausführungsform des erfindungsgemäßen Sklerosierungskatheters;
- Fig. 2 -: eine skizzenhafte Darstellung einer zweiten Ausführungsform des erfindungsgemäßen Sklerosierungskatheters;
- Fig. 3 -: eine skizzenhafte Darstellung einer dritten Ausführungsform des erfindungsgemäßen Sklerosierungskatheters;
- Fig. 4 -: eine skizzenhafte Darstellung eines vierten Ausführungsbeispieles eines erfindungsgemäßen Sklerosierungskatheters;
- Fig. 5 -: einen Querschnitt eines fünften Ausführungsbeispieles eines erfindungsgemäßen Sklerosierungskatheters;
- Fig. 6 -: einen Querschnitt eines sechsten Ausführungsbeispieles eines erfindungsgemäßen Sklerosierungskatheters;
- Fig. 7 -: einen Querschnitt eines siebten Ausführungsbeispieles eines erfindungsgemäßen Sklerosierungskatheters und
- Fig. 8 -: eine skizzenhafte Darstellung eines Axialschnittes durch den Sklerosierungskatheter gemäß Fig. 4.

In den Figuren der Zeichnung sind gleiche bzw. sich entsprechende Bauteile mit den gleichen Bezugszeichen versehen.

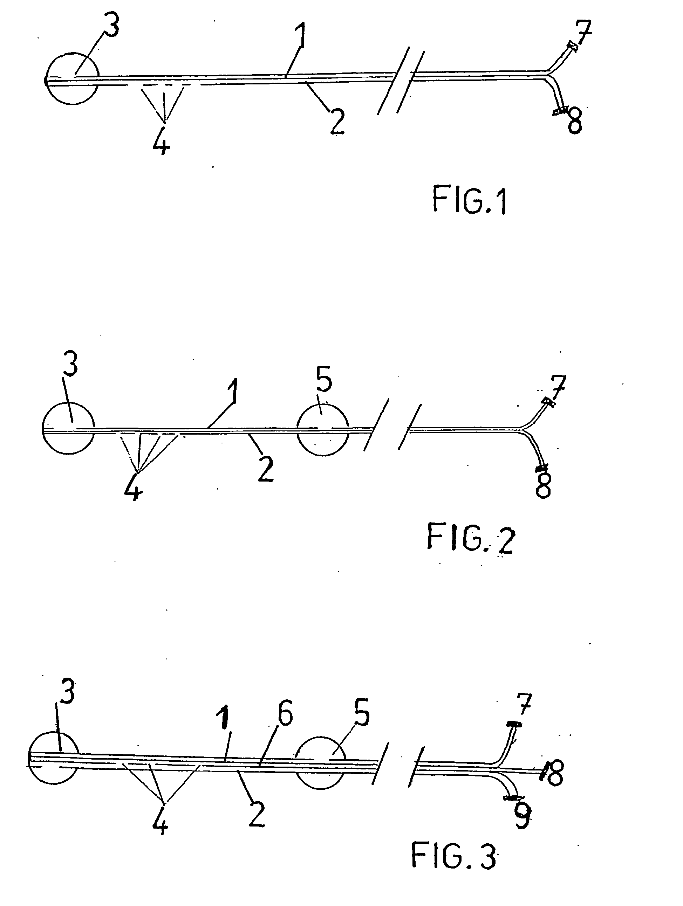

In Fig. 1 ist eine erste Ausführungsform des erfindungsgemäßen Sklerosierungskatheters skizzenhaft dargestellt. Der Sklerosierungskatheter weist ein erstes schlauchförmiges Lumen 1 auf, das an einem Ende über eine innere Öffnung mit einem ersten ballonartigen Element 3 verbunden ist, auf. Der Sklerosierungskatheter mündet an seinem anderen Ende in eine schlauchförmige Öffnung 7, über die mittels einer spritzenartigen Vorrichtung zu applizierende Luft eingespeist werden kann.

Weiterhin weist der Sklerosierungskatheter ein zweites schlauchförmiges Lumen 2 auf, das vor dem ballonartigen Element 3 mehrere Austrittsöffnungen 4 inne hat. Am anderen Ende des schlauchförmigen Lumens 2 ist eine Öffnung 8 angeordnet, über die mittels einer spritzenartigen Einrichtung ein zu applizierendes Sklerosierungsmittel eingespeist werden kann, welches durch die Austrittsöffnungen 4 in eine Gefäßinnenwand tritt.

Fig. 2 zeigt eine skizzenhafte Darstellung einer zweiten Ausgestaltung des erfindungsgemäßen Sklerosierungskatheters.

Dieser weist im Gegensatz zum in Fig. 1 gezeigten Sklerosierungskatheter ein zweites ballonartiges Element 5 auf, das mit dem schlauchförmigen Lumen 1 über eine innere Öffnung kommuniziert.

Fig. 3 zeigt skizzenhaft eine dritte Ausgestaltung des erfindungsgemäßen Sklerosierungskatheters.

Dieser Sklerosierungskatheter weist drei schlauchförmige Lumina 1, 2, 6 auf. Lumen 1 kommuniziert über eine innere Öffnung mit dem ballonartigen Element 5, während Lumen 2 über eine innere Öffnung mit dem ballonartigen Element 3 kommuniziert. Das dritte Lumen 6 weist zwischen den beiden ballonartigen Elementen 3, 5 mehrere Austrittsöffnungen 4 auf. Alle drei Lumina 1, 2, 6 sind unabhängig voneinander mit Luft bzw. einem Sklerosierungsmittel befüllbar (Lumen 1 und Lumen 2 mit Luft, Lumen 6 mit dem Sklerosierungsmittel). An den Enden der Lumina 1, 2, 6 sind Öffnungen 7, 9, 8 angeordnet, über die mittels einer spritzenartigen Einrichtung zu applizierende Luft (über die Öffnungen 7 und 9) bzw. ein zu applizierendes Sklerosierungsmittel (über die Öffnung 8) eingespeist werden kann. Vorteil dieser zwar im Vergleich zu den beiden vorherigen Ausführungsformen etwas aufwendigeren Konstruktion dieses Katheters ist die Tatsache, daß die beiden ballonartigen Elemente 3 und 5 unabhängig voneinander aufgeblasen werden können. Dies ermöglicht bei schwierigen Anwendungen eine feinfühlige Anpassung an die jeweiligen physiologischen Gegebenheiten.

Um ein Einführen des Sklerosierungskatheters auch in gekrümmte Blutgefäße zu ermöglichen, ist der Sklerosierungskatheter flexibel ausgebildet. Auf diese Weise paßt sich der Sklerosierungskatheter beim Einführen in das Blutgefäß in gewissen Grenzen an den Verlauf des Blutgefäßes an, so daß das Einführen in das Blutgefäß erleichtert ist. Erfindungsgemäß ist das zweite Lumen im wesentlichen entlang seiner gesamten Länge derart formstabil ausgebildet, daß bei Erzeugung eines zum Absaugen überschüssigen Sklerosierungsmittels aus der Vene erforderlichen Unterdruckes in dem zweiten Lumen ein Verschließen des zweiten Lumens durch Kontakt der Innenwandungen des zweiten Lumens miteinander verhindert ist.

Erfindungsgemäß sind die Austrittsöffnungen 4 relativ zu dem ersten ballonartigen Element 3 ortsfest angeordnet und in dem schlauchförmigen Lumen 2 gebildet. Da die Austrittsöffnungen 4 in dem schlauchförmigen Lumen 2 gebildet sind, das zwar flexibel, jedoch verhältnismäßig formstabil ausgebildet ist, besteht bei dem erfindungsgemäßen Katheter die Möglichkeit, nach Abschluß der Behandlung überschüssiges Sklerosierungsmittel aus dem Blutgefäß abzusaugen. Auf diese Weise ist verhindert, daß überschüssiges Sklerosierungsmittel in dem Butgefäß verbleibt. Das Absaugen des Sklerosierungsmittels kann beispielsweise dadurch erfolgen, daß ein Kolben der spritzenartigen Einrichtung zurückgezogen wird, wobei Luft aus dem Lumen 2 abgezogen und das Sklerosierungsmittel in das Lumen 2 hineingesaugt wird.

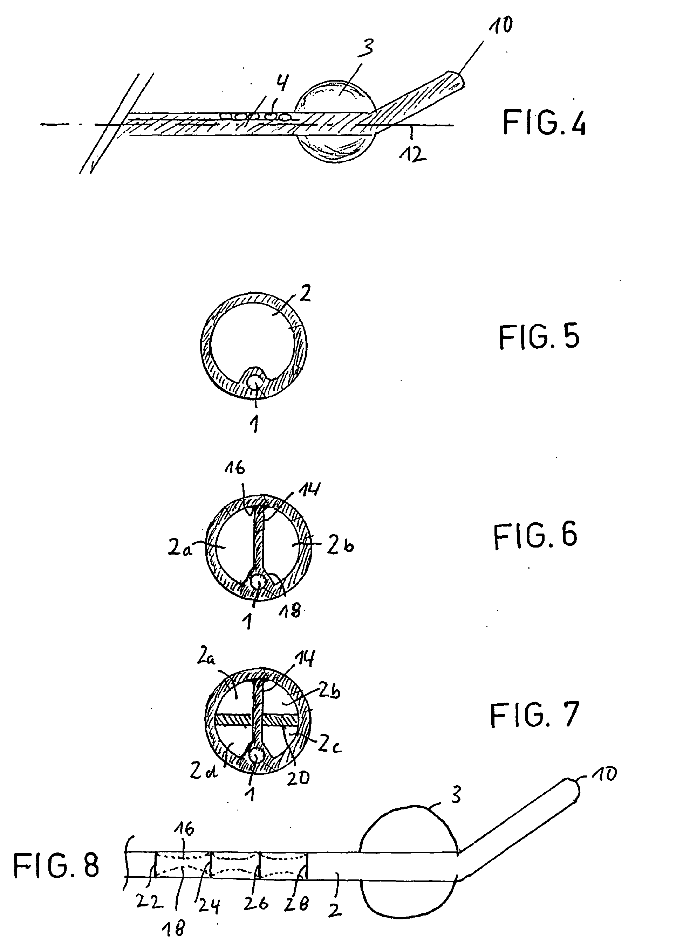

Fig. 4 zeigt skizzenhaft ein viertes Ausführungsbeispiel eines erfindungsgemäßen Sklerosierungskatheters, das sich von dem Ausführungsbeispiel gemäß Fig. 1 dadurch unterscheidet, daß das distale Ende 10 des Sklerosierungskatheters, der nachfolgend kurz als Katheter bezeichnet wird, unter einem Winkel von etwa 30° gegenüber der in Fig. 4 durch eine strichpunktierte Linie 12 symbolisierten Längsachse des Katheters abgebogen ist. Das distale Ende 10 des Katheters kann hierbei abgerundet sein, um eine Verletzung der Innenwandung des Blutgefäßes zu verhindern. Die Ausführungsform gemäß Fig. 4 hat den Vorteil, daß ein Einführen des Katheters in das Blutgefäß erleichtert ist. Stößt das distale Ende 10 des Katheters beim Einführen in das Blutgefäß an ein Hindernis, so kann der Katheter um seine Längsachse 12 in eine Verdrehlage verdreht werden, in der das distale Ende 10 nicht mehr an das Hindernis anstößt. Beispielsweise kann der Katheter so verdreht werden, daß die Abwinkelung an dem distalen Ende 10 dem Verlauf des Blutgefäßes folgt.

Der Winkel, unter dem das distale Ende 10 des Katheters abgewinkelt ist, ist in weiten Grenzen wählbar. Entsprechend den jeweiligen Anforderungen kann das distale Ende 10 des Katheters auch abgebogen ausgebildet sein.

Fig. 5 stellt einen Querschnitt eines fünften Ausführungsbeispieles eines erfindungsgemäßen Katheters dar, bei dem das zweite Lumen einen wesentlich größeren Querschnitt aufweist als das erste Lumen 1. Das erste Lumen 1 und das zweite Lumen 2 sind einstückig in einem Schlauchteil ausgebildet. Durch die einstückige Ausbildung beider Lumina 1, 2 ist die Formstabilität des zweiten Lumens 2 in dem Bereich, in dem das erste Lumen 1 angeordnet ist, erhöht.

Fig. 6 zeigt den Querschnitt eines sechsten Ausführungsbeispieles eines erfindungsgemäßen Katheters, das sich von dem Ausführungsbeispiel gemäß Fig. 5 dadurch unterscheidet, daß zur Erhöhung der Formstabilität des zweiten Lumens 2 Stabilisierungsmittel vorgesehen sind, die bei dem Ausführungsbeispiel gemäß Fig. 6 durch eine Rippe 14 gebildet ist, die sich in Radialrichtung des zweiten Lumens 2 zwischen den gegenüberliegenden Bereichen 16, 18 der Innenwandung des zweiten Lumens erstreckt. Aus der Zeichnung ist nicht ersichtlich und deshalb wird hier erläutert, daß sich die Rippe 14 in Axialrichtung des zweiten Lumens 2 über die gesamte Länge des zweiten Lumens 2 erstreckt, so daß das zweite Lumen 2 in zwei Einzellumina 2a, 2b unterteilt ist. Die Einzellumina 2a, 2b können entsprechend den jeweiligen Anforderungen voneinander getrennt sein oder miteinander kommunizieren. Durch die Rippe 14 ist die Formstabilität des zweiten Lumens 2 erhöht, so daß bei einem in dem zweiten Lumen 2 beim Absaugen überschüssigen Sklerosierungsmittels auftretenden Unterdruck eine Verformung des zweiten Lumens in Radialrichtung in Richtung der Rippe verhindert oder zumindest verringert ist.

Durch Unterteilung des zweiten Lumens 2 in zwei Einzellumina 2a, 2b ist ein dreilumiger Katheter gebildet. Hierbei kann eines der Einzellumina 2a, 2b beispielsweise zum Absaugen des Sklerosierungsmittels dienen, während über das andere Einzellumen beispielsweise eine Kochsalzlösung zugeführt und in das Blutgefäß eingebracht werden kann.

Fig. 7 zeigt den Querschnitt eines siebten Ausführungsbeispieles eines erfindungsgemäßen Katheters, das sich von dem Ausführungsbeispiel gemäß Fig. 6 dadurch unterscheidet, daß zusätzlich zu der Rippe 14 eine weitere Rippe 20 vorgesehen ist, die ebenfalls in Radialrichtung des zweiten Lumens 2, jedoch senkrecht zu der ersten Rippe 14 verläuft. Durch die Rippen 14, 20 ist bei Erzeugung eines Unterdruckes in dem zweiten Lumen 2 sowohl in Richtung der Rippe 14 als auch in Richtung der Rippe 20 eine Zusammendrückung des zweiten Lumens 2 verhindert oder zumindest verringert. Auf diese Weise ist besonders zuverlässig verhindert, daß sich das zweite Lumen 2 bei Erzeugung eines Unterdruckes durch Kontakt der Innenwandungen 16, 18 des zweiten Lumens 2 miteinander verschließt. Durch die Rippen 14, 20 ist das zweite Lumen 2 in vier Einzellumina 2a, 2b, 2c und 2d unterteilt, die entsprechend den jeweiligen Anforderungen miteinander kommunizieren oder voneinander getrennt sein können.

In Fig. 8 ist ein achtes Ausführungsbeispiel eines erfindungsgemäßen Katheters dargestellt, das sich von dem Ausführungsbeispiel gemäß Fig. 6 dadurch unterscheidet, daß anstelle der sich in Axialrichtung des zweiten Lumens 2 im wesentlichen über die gesamte Länge des zweiten Lumens erstreckenden Rippe 14 eine Mehrzahl von in Axialrichtung des zweiten Lumens zueinander beabstandeten Stegen 22, 24, 26, 28 vorgesehen ist. Die Stege 22, 24, 26, 28 können den gleichen Querschnitt aufweisen wie die Rippe 14 gemäß Fig. 6 bzw. die Rippen 14, 16 gemäß Fig. 7. Sie verhindern, daß bei Erzeugung eines Unterdruckes in dem zweiten Lumen 2 einander gegegenüberliegende Wandungsbereiche des zweiten Lumens 2 aneinander zur Anlage gelangen. Wird in dem zweiten Lumen 2 ein Unterdruck erzeugt, so verformt sich das zweite Lumen 2 an den axialen Stellen, an denen die Stege 22, 24, 26, 28 angeordnet sind, nicht oder nur wenig. In dazwischen liegenden axialen Bereichen verformen sich die Wandungen des zweiten Lumens 2 elastisch, wobei das Material der Wandungen des zweiten Lumens 2 und das Material der Stege 22, 24, 26, 28 sowie der axiale Abstand der Stege 22, 24, 26, 28 zueinander so gewählt sind, daß bei dem größten auftretenden Unterdruck ein Kontakt gegenüberliegender Bereiche 16, 18 der Innenwandung verhindert ist, wie in Fig. 8 durch gestrichelte Linien dargestellt.

## Patentansprüche

1. Sklerosierungskatheter zur Sklerosierung von Blutgefäßen, insbesondere Venen, mit mindestens einem ersten Lumen (1) und einem zweiten Lumen (2), wobei zum Stoppen des Blutflusses das erste Lumen (1) ein mit Luft befüll- und aufblasbares erstes ballonartiges Element (3) aufweist,
**dadurch gekennzeichnet,**
**daß** das zweite Lumen (2) mindestens eine relativ zum ersten ballonartigen Element (3) ortsfeste Austrittsöffnung (4) aufweist, aus der bei Benutzung des Sklerosierungskatheters Sklerosierungsmittel austritt, und
**daß** das zweite Lumen (2) im wesentlichen entlang seiner gesamten Länge derart formstabil ausgebildet ist, daß bei Erzeugung eines zum Absaugen überschüssigen Sklerosierungsmittels aus dem Blutgefäß erforderlichen Unterdruckes in dem zweiten Lumen (2) ein Verschließen des zweiten Lumens (2) durch elastische Verformung der Innenwandungen (16, 18) des zweiten Lumens (2) verhindert ist.

2. Sklerosierungskatheter nach Anspruch 1, **dadurch gekennzeichnet, daß** das erste Lumen (1) zum weiteren Verschließen des Blutgefäßes ein relativ zur Austrittsöffnung (4) beabstandetes, mit Luft befüll- und aufblasbares zweites ballonartiges Element (5) aufweist, wobei die Austrittsöffnung (4) zwischen erstem (3) und zweitem (5) ballonartigen Element angeordnet ist.

3. Sklerosierungskatheter nach Anspruch 1, **dadurch gekennzeichnet, daß** ein drittes Lumen (6) zum weiteren Verschließen des Blutgefäßes ein relativ zur Austrittsöffnung (4) beabstandetes, mit Luft befüll- und aufblasbares zweites ballonartiges Element (5) aufweist, wobei die Aus-trittsöffnung (4) zwischen erstem (3) und zweitem (5) ballonartigen Element angeordnet ist.

4. Sklerosierungskatheter nach einem der Ansprüche 1 bis 3, **dadurch gekennzeichnet, daß** das erste und/oder dritte Lumen (1, 6) eine Luftabsperreinrichtung (7) zum Absperren aus dem Lumen austretbarer Luft aufweist.

5. Sklerosierungskatheter nach Anspruch 4, **dadurch gekennzeichnet, daß** die Luftabsperreinrichtung ein Ventil, Gummistopfen oder eine Quetschklemme ist.

6. Sklerosierungskatheter nach Anspruch 1, **dadurch gekennzeichnet, daß** das distale Ende (10) des Sklerosierungskatheters abgebogen oder unter einem Winkel von weniger als 45°, vorzugsweise von etwa 30° zur Längsachse (12) des Sklerosierungskatheters abgewinkelt ist.

7. Sklerosierungskatheter nach Anspruch 1 oder 3, **dadurch gekennzeichnet, daß** die Wandungen des ersten Lumens (1) und/oder des zweiten Lumens (2) und/oder des dritten Lumens (6) wenigstens abschnittsweise aus einem elastisch verformbaren Material bestehen.

8. Sklerosierungskatheter nach Anspruch 1, **gekennzeichnet durch** Stabilisierungsmittel zur Formstabilisierung des zweiten Lumens (2).

9. Sklerosierungskatheter nach Anspruch 8, **dadurch gekennzeichnet, daß** die Stabilisierungsmittel wenigstens eine sich in Radialrichtung zwischen den Innenwandungen (16, 18) des zweiten Lumens erstreckende Rippe (14, 20) oder wenigstens einen sich in Radialrichtung zwischen den Innenwandungen (16, 18) des zweiten Lumens (2) erstreckenden Steg aufweisen.

10. Sklerosierungskatheter nach Anspruch 9, **dadurch gekennzeichnet, daß** sich die Rippe oder die Rippen (14, 20) bzw. der Steg oder die Stege in Axialrichtung im wesentlichen über die gesamte Länge des zweiten Lumens (2) erstreckt bzw. erstrecken.

11. Sklerosierungskatheter nach Anspruch 9, **dadurch gekennzeichnet, daß** eine Mehrzahl von Rippen oder Stegen (22, 24, 26, 28) vorgesehen ist, die sich jeweils über ein kurzes Stück der Länge des zweiten Lumens (2) erstrecken und die in Axialrichtung zueinander beabstandet sind.

12. Sklerosierungskatheter nach Anspruch 10, **dadurch gekennzeichnet, daß** das zweite Lumen durch die Rippe oder die Rippen (14, 20) bzw. den Steg oder die Stege in wenigstens zwei Einzellumina (2a, 2b, 2c, 2d) unterteilt ist.

13. Sklerosierungskatheter nach Anspruch 12, **dadurch gekennzeichnet, daß** die Einzellumina (2a, 2b, 2c, 2d) voneinander getrennt sind.

14. Sklerosierungskatheter nach Anspruch 12, **dadurch gekennzeichnet, daß** die Einzellumina (2a, 2b, 2c, 2d) miteinander kommunizieren.

## Claims

1. Sclerosing catheter for sclerosing blood vessels, especially veins, comprising at least a first lumen (1) and a second lumen (2), wherein the first lumen (1) comprises a first balloon-like element (3) that can be filled with air and inflated to stop blood flow ,
**characterised in that**
the second lumen (2) has at least one outlet opening (4) that is fixed in relation to the first balloon-like element (3) from which opening sclerosing means exits during the use of the sclerosing catheter, and
**in that** the second lumen (2) is designed to have a stable shape along its entire length so that when generating the required low pressure in the second lumen (2) to suction excess sclerosing means out of the blood vessel, the second lumen (2) is prevented from closing by the elastic deformation of the interior walls (16, 18) of the second lumen (2).

2. Sclerosing catheter according to claim 1, **characterised in that** for further closing the blood vessel the first lumen (1) has a second balloon-like element (5) that can be filled with air and inflated and which is spaced apart from the outlet opening (4), wherein the outlet opening (4) is arranged between the first (3) and second (5) balloon-like element.

3. Sclerosing catheter according to claim 1, **characterised in that** a third lumen (6) for closing the blood vessel further has a second balloon-like element (5) which is spaced apart from the outlet opening (4) and can be filled and inflated with air, wherein the outlet opening (4) is arranged between the first (3) and second (5) balloon-like element.

4. Sclerosing catheter according to one of claims 1 to 3, **characterised in that** the first and/or third lumen (1, 6) comprises an air blockage device (7) for blocking air escaping from the lumen.

5. Sclerosing catheter according to claim 4, **characterised in that** the air blockage device is a valve, a rubber plug or a compression clamp.

6. Sclerosing catheter according to claim 1, **characterised in that** the distal end (10) of the sclerosing catheter is bent or angled at an angle of less than 45°, preferably about 30°, to the longitudinal axis (12) of the sclerosing catheter.

7. Sclerosing catheter according to claim 1 or 3, **characterised in that** the walls of the first lumen (1) and/or the second lumen (2) and/or the third lumen (6) are made at least in sections from an elastically deformable material.

8. Sclerosing catheter according to claim 1, **characterised by** stabilising means for stabilising the shape of the second lumen (2).

9. Sclerosing catheter according to claim 8, **characterised in that** the stabilizing means comprise at least one rib (14, 20) extending in radial direction between the inner walls (16, 18) of the second lumen or at least one web extending in radial direction between the inner walls (16, 18) of the second lumen (2).

10. Sclerosing catheter according to claim 9, **characterised in that** the rib or the ribs (14, 20) or the web or the webs extends or extend in axial direction essentially over the entire length of the second lumen (2).

11. Sclerosing catheter according to claim 9, **characterised in that** a plurality of ribs or webs (22, 24, 26, 28) is provided, which extend respectively over a short part of the length of the second lumen (2) and which are spaced apart from one another in axial direction.

12. Sclerosing catheter according to claim 10, **characterised in that** the second lumen is subdivided by the rib or the ribs (14, 20) or the web or webs into at least two individual lumens (2a, 2b, 2c, 2d).

13. Sclerosing catheter according to claim 12, **characterised in that** the individual lumens (2a, 2b, 2c, 2d) are separated from one another.

14. Sclerosing catheter according to claim 12, **characterised in that** the individual lumens (2a, 2b, 2c, 2d) communicate with one another.

## Revendications

1. Cathéter sclérosant destiné à scléroser des vaisseaux sanguins, en particulier des veines, avec au moins un premier lumen (1) et un deuxième lumen (2), le premier lumen (1) comportant un premier élément en forme de ballon (3) remplissable et gonflable à l'air pour arrêter la circulation sanguine,
**caractérisé**
**en ce que** le deuxième lumen (2) comporte au moins un orifice de sortie (4) fixe par rapport au premier élément en forme de ballon (3), par où s'écoule l'agent sclérosant lors de l'utilisation du cathéter sclérosant, et
**en ce que** le deuxième lumen (2) est réalisé avec une stabilité de forme sensiblement sur toute sa longueur, telle qu'en cas de génération dans le deuxième lumen (2) d'une dépression exigée pour l'aspiration de l'agent sclérosant excédentaire dans le vaisseau sanguin, une obturation du deuxième lumen (2) par déformation élastique des parois internes (16, 18) du deuxième lumen (2) est empêchée.

2. Cathéter sclérosant selon la revendication 1, **caractérisé en ce que** le premier lumen (1) comporte à distance de l'orifice de sortie (4) un deuxième élément en forme de ballon (5) remplissable et gonflable à l'air pour une obturation complémentaire du vaisseau sanguin, l'orifice de sortie (4) étant disposé entre le premier (3) et le deuxième (5) éléments en forme de ballon.

3. Cathéter sclérosant selon la revendication 1, **caractérisé en ce qu'**un troisième lumen (6) comporte à distance de l'orifice de sortie (4) un deuxième élément en forme de ballon (5) remplissable et gonflable à l'air pour une obturation complémentaire du vaisseau sanguin, l'orifice de sortie (4) étant disposé entre le premier (3) et le deuxième (5) éléments en forme de ballon.

4. Cathéter sclérosant selon l'une des revendications 1 à 3, **caractérisé en ce que** le premier et/ou le troisième lumen (1, 6) comporte un dispositif d'arrêt d'air (7) pour l'arrêt de l'air pouvant sortir du lumen.

5. Cathéter sclérosant selon la revendication 4, **caractérisé en ce que** le dispositif d'arrêt d'air est une valve, un bouchon en caoutchouc ou une pince de serrage.

6. Cathéter sclérosant selon la revendication 1, **caractérisé en ce que** l'extrémité distale (10) du cathéter sclérosant est pliée ou coudée suivant un angle inférieur à 45 °, de préférence d'environ 30 ° par rapport à l'axe longitudinal (12) du cathéter sclérosant.

7. Cathéter sclérosant selon la revendication 1 ou la revendication 3, **caractérisé en ce que** les parois du premier lumen (1) et/ou du deuxième lumen (2) et/ou du troisième lumen (6) sont au moins partiellement composées d'un matériau élastiquement déformable.

8. Cathéter sclérosant selon la revendication 1, **caractérisé par** des moyens de renforcement pour la stabilité de forme du deuxième lumen (2).

9. Cathéter sclérosant selon la revendication 8, **caractérisé en ce que** les moyens de renforcement comportent au moins une nervure (14, 20) s'étendant en direction radiale entre les parois internes (16, 18) du deuxième lumen, ou au moins une cloison s'étendant en direction radiale entre les parois internes (16, 18) du deuxième lumen (2).

10. Cathéter sclérosant selon la revendication 9, **caractérisé en ce que** la nervure ou les nervures (14, 20), ou la cloison ou les cloisons s'étendent en direction axiale sensiblement sur toute la longueur du deuxième lumen (2).

11. Cathéter sclérosant selon la revendication 9, **caractérisé en ce qu'**une pluralité de nervures ou de cloisons (22, 24, 26, 28) sont prévues, lesquelles s'étendent chacune sur une faible partie de la longueur du deuxième lumen (2) et sont espacées entre elles en direction axiale.

12. Cathéter sclérosant selon la revendication 10, **caractérisé en ce que** le deuxième lumen est divisé en au moins deux lumens isolés (2a, 2b, 2c, 2d) par la nervure ou les nervures (14, 20), ou la cloison ou les cloisons.

13. Cathéter sclérosant selon la revendication 12, **caractérisé en ce que** les lumens isolés (2a, 2b, 2c, 2d) sont séparés entre eux.

14. Cathéter sclérosant selon la revendication 12, **caractérisé en ce que** les lumens isolés (2a, 2b, 2c, 2d) communiquent entre eux.
